# EUROPEAN PATENT SPECIFICATION

(11) **EP 3 539 609 B1**
(45) Date of publication and mention of the grant of the patent: **28.04.2021**
(21) Application number: 18162283.8
(22) Date of filing: 16.03.2018
(51) Int. Cl.: A61N 1/36

(54) **VERSATILE CONTROL FOR A NEURAL STIMULATION DEVICE**
VIELSEITIGE STEUERUNG FÜR EINE NERVENSTIMULATIONSVORRICHTUNG
COMMANDE POLYVALENTE POUR DISPOSITIF DE STIMULATION NEURONALE

(43) Date of publication of application: 18.09.2019
(73) Proprietor: Universität Ulm, 89081 Ulm (DE)
(72) Inventor: Ortmanns, Prof. Dr.-Ing. Maurits, 89075 Ulm (DE); Haas, Michael, 88400 Biberach (DE)
(74) Representative: Pfenning, Meinig & Partner mbB

(56) References cited:
- US-A1- 2008 079 406
- US-A1- 2009 048 643
- US-A1- 2014 277 269

## Description

The invention relates to a control device for a neural stimulation device comprising an electrode voltage connector configured to be connected to the neural stimulation device for obtaining an electrode voltage of the neural stimulation device and a current control signal connector configured to be connected to the neural stimulation device for providing a current control signal to the neural stimulation device, where an electrode current of the neural stimulation device is controlled by the current control signal, as well as to a corresponding method for operating a control device for a neural stimulation device.

State-of-the-art neural stimulators can be divided into three categories, constant voltage stimulators (CVS), constant current stimulators (CCS) and constant charge stimulators.

The more recent and rather exotic charge controlled stimulators apply a programmable amount of charge to the working electrode. This is usually achieved by using switched capacitor stimulation circuits [1]. The disadvantage is the need for very large charging capacitors, which make the technique rather infeasible for high channel count neural interfaces.

In current controlled stimulators (CCS), which dominate the state of the art in integrated stimulators nowadays, a programmable current waveform is used as stimulation signal. Together with precise timing, this method ensures, that a well-defined amount of charge is applied to the nervous tissue, causing the desired stimulation effect [2].

The third method, controlled voltage stimulation (CVS), allows to define a voltage waveform, which is then applied to the working electrode [4]. Although this method can cause severe safety issues it is still very popular among researchers and clinicians, as large amounts of data, equipment and experience exist from former work and since this stimulation is the most power efficient.

Commonly, CVS and CCS stimulators are independently realized. Until now, there are only very few publications, which combine a voltage and current mode stimulator [5,7,8]. In one implementation, an analog, high voltage (HV) transimpedance amplifier (TIA) is used to generate a stimulation voltage out of an adjustable reference current. In CCS mode the current is directed to the anodic or cathodic branch of a HV output current stage. In CVS mode the current is fed into a resistive feedback HV TIA, which generates the desired output voltage.

Actually, such a reconfigurable circuit, which combines both functionalities with reuse of the existing circuitry, has only been shown once in the state of the art [5]. Said reconfigurable CVS and CCS stimulator uses a resistive-feedback operational amplifier, which acts as a TIA. In current mode, first and second transistors act as output current source and provide the stimulation current. In the voltage mode, the first transistors are reused, to form the output stage of the operational amplifier, together with third transistors. Other implementations do very similar implementations or architectures, which rely on discrete components which are arranged to setup the reconfigurable CCS or CVS modes.

A new architecture for a pure current controlled stimulator was proposed in [3], which uses a semi-digital feedback loop in order to control the stimulation current steered into a stimulation electrode. This implementation allows a precise monitoring of the stimulation current in order to precisely control the amount of charge injected into the tissue.

Nonetheless, the architecture in [3] generally comes with two major drawbacks. First, the input of the HV transimpedance amplifier (TIA), which is used to monitor the stimulation current, is directly connected to the working electrode leading to stability problems. The second problem is, that the two TIAs require a constant biasing current in the HV domain, which leads to a poor power efficiency.

To be mentioned at this point is a technique - principally unrelated to the present invention - which is called charge balancing. This is needed since the delivered charge to stimulation electrodes is never 100% balanced over time, meaning that a net charge transfer to the electrode can not be avoided due to stimulator imperfections.

A charge balancer is now used to steer the electrode voltage after completion of a single stimulation pulse or a multitude of stimulation pulses back into a safe voltage window such as to prevent irreversible electrochemical reactions at the electrode. One of those circuits has been patented in [6], with the main claim: "an electrical circuit which controls the electric voltage or the electric charge on the stimulating electrodes as a function of the electric voltage between the stimulating electrodes and reduces or equalizes imbalances of electric charges on the stimulating electrodes".

Most of the state of the art stimulator implementations have the drawback, that only one of the three above mentioned stimulation modes is implemented. Thus, the user is not flexible in the application. Therefore, currently available devices lack the possibility to compare fundamentally different stimulation methods and their efficacy using the same device.

The combined and reconfigurable CCS/CVS stimulation device presented in [5] offers the possibility of switching between voltage and current mode stimulation, but has a bad power efficiency in voltage mode. Since a class A output stage is used, a constant bias current is necessary even when the output current is zero (in contrast, in a class B output stage, the bias current is or may be zero when the output current is zero). As the corresponding voltage drop is usually large (>10 V) in order to enable a large output voltage range, small biasing currents will already cause a large power dissipation.

The design presented in [3] suffers from the same biasing problem. Both TIAs require static currents from the HV supply, leading to poor power efficiency.

As implantable, biomedical stimulators usually require several, independent stimulation sites and come with a strict power limitation, this waste of power is not acceptable.

Moreover in the state of the art implementation in [3] and [5], the electrode is placed at the input of a HV differential amplifier (as part of the TIA). Especially capacitive loads at this point make the amplifier unstable, which results in frequency compensation in the TIA-feedback. This is widely known e.g. from photosensor readouts. Disadvantageously, in neural stimulator applications, the load at this point is the electrode itself, whose linear small signal model is a capacitor and a resistor, but both can easily vary over orders of magnitudes for different electrode geometries, materials and facing aging. Thus, the state of the art is very limited in the applicable stimulation electrodes for stability reasons.

Additionally, the HV input stage of the TIA in both designs is area consuming, since it requires large HV transistors, making the respective control devices cumbersome and expensive.
[1] H.-M. Lee, K.-Y. Kwon, W. Li, M. Ghovanloo, "A Power-Efficient Switched-Capacitor Stimulating System for Electrical/Optical Deep-Brain Stimulation", IEEE J. Solid-State Circuits, vol. 50, no. 1, pp. 360-374, Oct. 2014.
[2] M. Haas, U. Bihr, J. Anders, M. Ortmanns, "A bidirectional neural interface IC with high voltage compliance and spectral separation" IEEE International Symposium on Circuits and Systems (ISCAS), Montreal, 2016, pp. 2743-2746
[3] H. Kassiri, G. Dutta, N. Soltani, C. Liu, Y. Hu, R. Genov, "An Impedance-Tracking Battery-less Arbitrary-Waveform Neurostimulator with Load-Adaptive 20VVoltage Compliance", ESSCIRC 2016: 42nd European Solid-State Circuits Conference, Lausanne, 2016, pp. 225-228.
[4] F. Shahrokhi, K. Abdelhalim, D. Serletis, P. Carlen, and R. Genov, "The 128-channel fully differential digital integrated neural recording and stimulation interface," IEEE Transactions on Biomedical Circuits and Systems, vol. 4, no. 3, pp. 149 - 161, June 2010.
[5] N. Laotaveerungrueng, R. R. Lahiji, S. L. Garverick and M. Mehregany, "A high-voltage, high-current CMOS pulse generator ASIC for deep brain stimulation", 2010 Annual International Conference of the IEEE EMBS, Buenos Aires 2010, pp.1519-1522
[6] A. Rocke, N. Unger, M. Ortmanns, Vorrichtung zur steuerung der elektrischen ladung an stimulationseletroden, EP 1827591 B1. Similar:
[7] Anthony Guilvard ; Amir Eftekhar; Song Luan ; Christofer Toumazou ; Timothy G. Constandinou, A fully-programmable neural interface for multi-polar, multi-channel stimulation strategies, Circuits and Systems (ISCAS), 2012 IEEE International Symposium on.
[8] Daniel A Wagenaar and Steve M Potter, A versatile all-channel stimulator for electrode arrays, with real-time control, 15 March 2004 • 2004, Journal of Neural Engineering, Volume 1, Number 1.

US 2014/0277269 A1 discloses a successive approximation analog-to-digital converter made of a low voltage configurable differential amplifier and low voltage logic circuits which can convert a high voltage analog input to a digital equivalent. This arrangement provides for the measurement of large electrode voltages by first attenuating the high electrode voltage range to a lower voltage range across a capacitor.

US 2009/0048643 A1 describes a method of operating an implantable pulse generator comprising the steps of: providing power to a voltage converter at a first voltage level, outputting a second voltage level by the voltage converter, the second voltage level being a variable voltage level that is controlled by a control signal provided to the voltage converter, the voltage level being provided to pulse generating circuitry of the implantable pulse generator, the second voltage level being selectable from a plurality of voltages including non-integer multiples of the first voltage level, generating pulses by the pulse generating circuitry, the pulse generating circuitry including current control circuitry for controlling the pulses to cause the pulses to provide substantially constant current to tissue of the patient, and applying at least two different control signals to the voltage converter during individual pulses to provide successively increasing voltages to the pulse generating circuitry during a respective current pulse.

It is hence an objective of the present invention to provide a power-efficient control for a versatile and reliable use of a neural simulation device.

This objective is achieved by the subject-matter of the independent claims. Advantageous embodiments are disclosed by the dependent claims, the description, and the figures.

An aspect of the invention relates to a control device for a neural stimulation device which is configured to be used to generate stimulation signals for neural tissues, e.g. for brain machine interfaces and/or functional electrical stimulation of neurons. Such a control device comprises an electrode voltage connector (also referred to as terminal or port), which is configured to be connected to the neural stimulation device for obtaining an electrode voltage of the neural stimulation device, and a current control signal connector, which is configured to be connected to the neural stimulation device for providing a current control signal to the neural stimulation device. Therein, the current control signal is suited to control an electrode current of the neural stimulation device. In particular, the control device is configured to provide the current control signal while the electrode voltage is obtained.

The control device further comprises an analog-to-digital converter (ADC) unit, which is configured to digitize an input voltage in dependence of (in particular: relative to) a preset (which is also to be understood in the sense of "presettable") reference voltage V_{ref} and to provide a digital output signal as a result of the digitization. The digitization can be done using single-bit resolution (comparator) or multibit resolution. In case of the single-bit resolution, the analog-to-digital converter unit acts as a comparator unit which compares the input voltage with the reference voltage. The input voltage is either the electrode voltage obtained via the electrode voltage connector or a converted voltage corresponding to the electrode voltage obtained via the electrode voltage connector. For instance, the digitized input voltage may be a fixed fractional amount of the electrode voltage or a dynamically adjustable fractional amount of the electrode voltage. The digitalization of the input voltage relative to the reference value may be a digitalization of the input voltage subtracted from the reference voltage and/or a digitalization of the difference between the input voltage and the reference value.

The control device also comprises a digital control unit, which is configured to generate the current control signal in dependence upon the digital output signal provided by the ADC unit and to provide the control signal to the current control signal connector. Therein, the control signal is a digital control signal. The control device may be configured to generate the current control signal based on the digital output signal provided by the ADC unit, that is, based on the digitization result, while the electrode voltage is obtained (or acquired). Hence, the digital control unit may be configured to generate the current control signal in real-time.

The idea underlying the invention may thus be summarized as follows: use a voltage measurement device at the electrode of the neural stimulation device to sense and digitize the difference between a (e.g. digitally) adjusted, desired stimulation reference voltage V_{ref} and the actual electrode voltage at the working electrode, add a digital control unit to achieve a wanted control behavior, and control the electrode voltage of the neural stimulation device towards an adjusted value using the already implemented HV current sources of the neural stimulation device. When the current value is (digitally) changed, the electrode voltage follows the digital control signal. Therein, the measurement device (or ADC unit) may be as simple as a 1-bit-comparator which uses a 1-bit analog-to-digital converter to compare electrode voltage with the preset reference voltage and decide if the electrode voltage or the reference voltage is greater. This is one example for digitizing the input voltage relative to the reference voltage. The measurement device may also use a multi-bit quantizer (ADC) for improved control behavior. Thus, reusing a conventional CCS neural stimulation device, the control device described above can be used to configure the conventional CCS neural stimulation device into a CVS mode with small, mostly digital circuit overhead.

The above control device provides high flexibility for a neural stimulation device as it can be digitally, i.e. simply, configured to provide constant current stimulation (CCS) or constant voltage stimulation (CVS) current control signals. Additionally, both configurations can allow the programming of arbitrary stimulation waveforms in the voltage or current domain. Hence the disclosed control device provides a versatile, integrated solution for a multitude of stimulation scenarios.

By reconfiguring the commonly employed current mode high-voltage (HV) output stage of a CCS neural stimulation device, the main part of the circuit of the neural stimulation device can be reused by the described control device for CVS. The described digitally controlled feedback loop with the control device is used in order to allow class B operation for voltage mode stimulation as well as current mode stimulation. Hence, no static biasing currents are required when the output current current is zero, in particular in the HV domain (compare below). This allows a highly area and power efficient implementation, with extremely small circuit overhead over the already implemented CCS stimulator.

The aforementioned low area and power consumption make the control device suitable for implantable, biomedical stimulators, which usually require HV stimulation signals but come with strict area and power limitations. Such systems are used for brain machine interfaces (BMI) or functional electrical stimulation (FES), to overcome lost or missing body functions.

In the presented control device, the stability of the control loop only requires a very small resistive part in the load at the output. Thus, the circuit can be used for various electrodes and would not require frequency compensation for largely variable electrode loads, as compared to the state of the art [3,5], for instance. The flexibility to choose between voltage and current mode stimulation allows researchers the direct comparison between the two stimulation types.

In an advantageous embodiment, the preset reference voltage is a dynamically preset reference voltage.

This gives the advantage that a complex control behavior of the control device can be realized, in particular a constant voltage stimulation or a stimulation with arbitrary stimulation waveforms.

In another advantageous embodiment, the preset reference voltage is selectable to be zero or, in addition, to be another preset reference voltage by a user. Therein, the other preset reference voltage selectable by the user may also be a dynamically changing reference voltage, for instance a reference voltage changing according to a preset scheme.

This gives the advantage of a secondary use in addition to the control of stimulation of the neural tissue. Namely the control device can also, even simultaneously during a stimulation, be used for the above mentioned charge balancing at stimulation electrodes. Due to circuit imperfections, the net charge delivered to an electrode is never exactly zero, and thus a residual voltage can build up at an electrode, even though charge balanced stimulation pulses (anodic+cathodic stimulation charges equal zero) have been applied. This residual voltage due to imbalanced stimulation can exceed safe limits, which would lead to strong Faradaic currents, electrode corrosion as well as tissue destruction by pH change.

The state of the art either performs passive balancing (based on shorting the electrode after stimulation towards e.g. ground, which thus eliminates the residual charge), which has the disadvantage of long discharge times, during which neither another stimulation can be run, nor the electrode can be used for recording of signals. Alternatively, the state of the art proposed active discharge, where the electrode residual charge is measured after stimulation and thereafter balancing charges are applied in order to discharge the electrode. Disadvantageously, it needs additional circuit overhead and high precision requires complex circuitry and a large space.

In the presented embodiment, after a current mode stimulation, the stimulator can be reconfigured to voltage mode, and the electrode can be steered towards a digitally defined voltage. This can be ground (zero), but could also be any other feasible voltage value, which can be adjusted using, for instance, a digital-to-analog converter for V_{ref} generation. The latter is advantageous, since a pre-biased electrode can have beneficial properties concerning charge storage capacity, corrosion, etc.

In yet another embodiment, the ADC unit comprises a 1-bit analog-to-digital converter which is used in digitizing the input voltage relative to the reference voltage, in particular to generate the digital output signal with the digitizing comprising comparing the input voltage with the reference voltage.

This gives the advantage that the ADC unit is particularly simple, robust, and small. As consequence, the control unit can be relatively simple, robust, and small as well, as the output signal may be a single bit or reflect the comparison result by a single bit.

In a preferred embodiment, the control device comprises a conversion unit configured to convert the electrode voltage obtained via the electrode voltage connector into the converted voltage, which then corresponds to the electrode voltage obtained via the electrode voltage connector, as input voltage for the ADC unit, where the converted voltage is lower than the electrode voltage. This preferred embodiment thus may comprise a HV to LV conversion, where HV may be any voltage of 6 Volt or higher, for instance, and LV may be any voltage of 5 Volt or lower, for instance. In particular, the converted voltage may be 5 Volt or lower, or 3 Volt or lower or 1,8 Volt or lower. The digitally adjustable reference voltage V_{ref} may, for instance, be provided by an on-chip digital-to-analog converter (DAC). As this DAC can be realized in the LV domain and needs only little driving capability, highly power and area efficient implementations can be realized.

This gives the advantage of a design in which as much signal processing as possible is done in a LV domain. Since the supply voltage in the HV domain must be large to provide sufficient output swing, already small currents cause large power consumption, leading to a bad stimulator efficiency for class-A stimulators, which is avoided in the proposed configuration. The decreased number of HV transistors reduces the area overhead, as the adjacent circuitry can be realized with miniaturized LV devices. Therefore the invention achieves very high area efficiency by minimizing the number of HV devices and high power efficiency by minimizing all HV biasing currents. Said voltage numbers allow for the use of small standardized components, increasing availability (and hence flexibility in the design) and robustness.

In comparison to the solution proposed in [5], the overhead circuitry and power consumption is reduced by the digital feedback loop. The digital signal processing allows class-B operation in current and voltage mode, eliminating the unwanted power consumption caused by biasing currents in the HV domain.

Compared to the semi-digital design proposed in [3], the invention is much more area efficient and requires again less power. By converting the voltage of the electrode into a discrete time, LV signal first, all adjacent parts can be realized in the LV domain. In [3] two HV differential amplifiers are needed, requiring large silicon area because of bulky HV transistors. The biasing current required for these amplifiers must again be provided over the large HV supply voltage, leading to bad power efficiency.

Additionally the designs proposed in [3] and [5] may suffer from stability problems under the case of large, capacitive loads, since the load is directly connected to the input of the transimpedance amplifier (TIA).

In a further advantageous embodiment, the conversion unit comprises a capacitive divider. In particular the conversion unit may comprise a capacitive divider with at least one variable capacity, such as a variable capacitor, so as to enable a variable voltage conversion.

This gives the advantage that a capacity-change instead of a voltage-change of the preset reference voltage V_{ref} can be used to achieve the desired behavior. As varying a capacity can be realized easier than varying a voltage, system complexity and power consumption is reduced and reliability enhanced.

In a particularly advantageous embodiment, the control device comprises a capacity divider being configured to periodically sample the electrode voltage. Furthermore, in this embodiment, the ADC unit (which may also be referred to as comparator unit) is configured to periodically sample the input voltage with the same period as the sampling of the electrode voltage and with a phase that is shifted, in particular delayed, with respect to the phase of the sampling of the electrode voltage.

This gives the advantage of a simple, integrated solution that allows the reconfiguration from CCS into CVS. By using a discrete time HV to LV converter only one additional HV switch transistor, a LV single- or multi-bit ADC together with a DAC and a digital filter are required. The decreased number of HV transistors reduces the area overhead, as the adjacent circuitry can be realized with miniaturized LV devices. By implementing the ADC as such a dynamic ADC unit or comparator, almost all biasing currents in the LV domains are eliminated as well. The shift or delay of the phase compensates for delays caused by the switches that are used for the sampling and hence increases the accuracy of the control.

Another aspect of the invention relates to a neural stimulation device with a control device according to any of the preceding embodiments.

A further aspect of the invention relates to a method for operating a control device for a neural stimulation device. Such a method may be a computer-implemented method and comprises a number of method steps. A first step is obtaining an electrode voltage of the neural stimulation device. Another step is digitizing an input voltage, which is the obtained electrode voltage or a converted voltage corresponding to the obtained electrode voltage, in dependence of a preset reference voltage (which may be or comprise comparing the input voltage with the reference voltage), where a subsequent step is providing a digital output signal as a result of the digitizing. Yet another step is generating a current control signal, which is to be used for controlling an electrode current of the neural stimulation device, in dependence upon the provided digital output signal. Another step is providing the current control signal to the neural stimulation device.

Advantages and advantageous embodiments of the method correspond to advantages and advantageous embodiments of the described control device.

The features and combinations of features described above, as well as the features and combinations of features disclosed in the figure description or the figures alone may not only be used alone or in the described combination, but also with other features or without some of the disclosed features without leaving the scope of the invention. Consequently, embodiments that are not explicitly shown and described by the figures but that can be generated by separately combining the individual features disclosed in the figures are also part of the invention. Therefore, embodiments and combinations of features that do not comprise all features of an originally formulated independent claim are to be regarded as disclosed. Furthermore, embodiments and combinations of features that differ from or extend beyond the combinations of features described by the dependencies of the claims are to be regarded as disclosed.

Exemplary embodiments are further described in the following by means of schematic drawings. Therein,
Fig. 1 shows a schematic wiring diagram of an exemplary control device with corresponding neural stimulation device;
Fig. 2 shows a schematic wiring diagram of another exemplary control device;
Fig. 3 shows an exemplary timing of the sampling phases for the control device of Fig. 2; and
Fig. 4 shows a schematic wiring diagram of yet another exemplary control device.

In the figures, identical or functionally identical elements have the same reference signs.

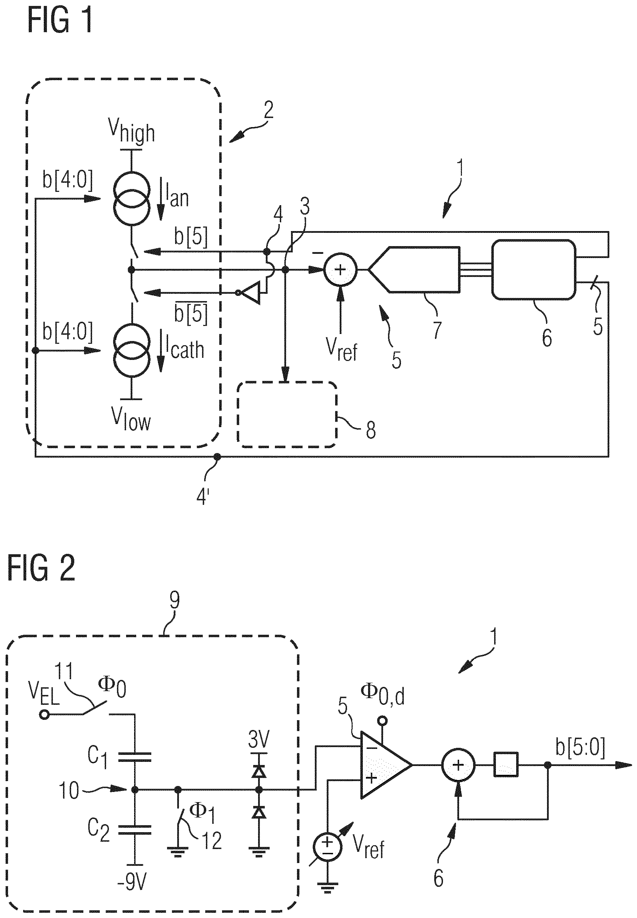

Figure 1 shows a schematic wiring diagram of an exemplary control device with corresponding neural stimulation device. Therein, the control device 1 for the neural stimulation device 2 comprises an electrode voltage connector 3 configured to be connected to the neural stimulation device 2 for obtaining an electrode voltage V_{EL} of the neural stimulation device 2, a current control signal connector 4, 4' configured to be connected to the neural stimulation device 2 for providing a current control signal b[5:0], b[5], b[4:0] to the neural stimulation device 2, where an electrode current of the neural stimulation device 2 is controlled by the current control signal b[5:0], b[5], b[4:0], as well as a analog-to-digital converter (ADC) unit 5 configured to digitize an input voltage, which is the electrode voltage V_{EL} obtained via the electrode voltage connector 3 or a converted voltage corresponding to the electrode voltage V_{EL} obtained via the electrode voltage connector 3, in dependence upon a preset reference voltage V_{ref} and provide a digital output signal as a result of the digitization, and a digital control unit 6 configured to generate the current control signal b[5:0], b[5], b[4:0] in dependence upon the digital output signal provided by the ADC unit 5 and provide the current control signal b[5:0], b[5], b[4:0] to the current control signal connector 4, 4'.

In the present example, the current control signal b[5:0], b[5], b[4:0] is a digital 6-bit signal, where the 6th bit b[5] is fed into the neural stimulation device 2 via the current control signal connector 4 to activate an anodic current Iₐₙ of a high potential V_{high} voltage source or a cathodic current I_{cath} of a low potential V_{low} voltage source in the neural stimulation device 2 for an attached electrode 8. The 1st to 5th bit b[4:0] is fed into the neural stimulation device 2 via the current control signal connector 4' to determine the level of the respective current Iₐₙ, I_{cath}. Generally, the control signal may be any digital n-bit signal, where, for instance, the nth bit b[n-1] may be fed into the neural stimulation device 2 via the current control signal connector 4 to activate an anodic current Iₐₙ of a high potential V_{high} voltage source or a cathodic current I_{cath} of a low potential V_{low} voltage source in the neural stimulation device 2 for an attached electrode 8 and the 1st to (n-1)th bit b[(n-2):0] may be fed into the neural stimulation device 2 via the current control signal connector 4' to determine the level of the respective current Iₐₙ, I_{cath}.

So, a voltage meter (advantageously implemented with an analog-to-digital converter (ADC)) may be used in the present example to sense the difference between a digitally adjusted, desired stimulation voltage V_{ref} and the actual voltage V_{EL} at the working electrode 8. The digitized value is then fed into a digital control unit 6 that may be a digital filter, whose exemplary 6 bit output b[5:0] controls the bidirectional current controlled stimulator 2.

Figure 2 shows a schematic wiring diagram of another exemplary control device. In that example, the control device 1 comprises a conversion unit 9 configured to convert the HV electrode voltage V_{EL} into a lower LV input voltage for the ADC unit 5. In the present example, the conversion unit 9 features a capacitive divider 10 to convert the HV voltage into the LV voltage and the ADC unit 5 is implemented as a single-bit-comparator. Here, the capacitive divider 10 has a first capacity C₁ and a second capacity C₂, which may be n times as big as C₁ in order to reduce the HV voltage with a ratio of 1:(n+1), that is, 1:6 for instance. This allows to convert an electrode voltage V_{EL} in the range from ±9V into an input voltage for the ADC unit 5 in the range from 0-3V. Again, the values of (±)9V and 3V depicted in the figure are exemplary values that can be replaced by any suitable other value.

In the shown example, the capacitive divider 10 (in general, this may be accomplished by the conversion unit 9) is configured to periodically sample the electrode voltage V_{EL} by closing and opening a first switch 11 and connecting and disconnecting the capacities C1, C2 to the electrode voltage V_{EL}, respectively, with a sampling phase Φ₀. By grounding the capacities C₁, C₂ with closing and opening a second switch 12 periodically with a phase Φ₁, the desired input voltage for the ADC unit 5 is obtained.

The described preferred implementation includes a HV to LV conversion, the (e.g. single bit) ADC, and the digital filter or control unit 6. Starting from the electrode potential on the left hand side in Fig. 2, e.g. a capacitive divider 10 can be used to reduce the HV electrode voltage V_{EL}, e.g. with a ratio of 1:6, in order to convert an electrode voltage from e.g. a ±9V range into a digital supply voltage range of e.g. 0-3V. This divider 10 samples the electrode voltage V_{EL} on phase Φ₀.

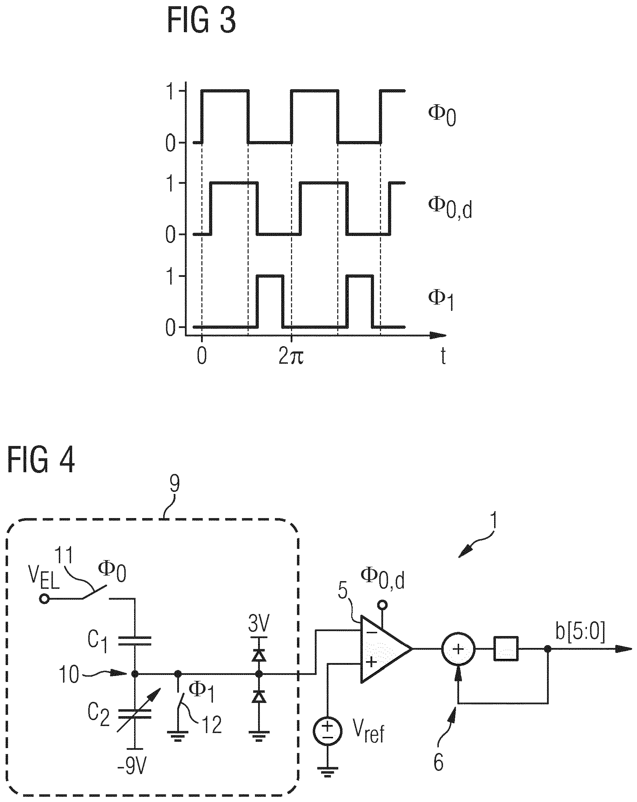

The (preferably digitally) adjustable reference voltage V_{ref} may be provided by an on-chip digital-to-analog converter (DAC). As this DAC can be realized in the LV domain and needs only little driving capability, highly power and area efficient implementations can be realized. Alternatively to the adjustable reference voltage V_{ref}, the reference voltage V_{ref} may be fixed and the first capacity C₁ and/or second capacity C₂ may be varied. Yet another exemplary control device which realizes said alternative with a fixed reference voltage V_{ref} and with a variable capacity, a variable second capacity C₂ in this case, is shown in Figure 4.

So, advantageously, for low power and area consumption, the dynamic ADC or comparator unit 5 performs the analog-to-digital conversion, where the ADC or comparator is triggered by a delayed version of the sampling phase Φ₀, as shown in the timing diagram in Figure 3. The negative summation node of Fig. 1 may be realized by applying the output of the DAC V_{ref} to the positive input terminal of the ADC 5 and the sampled electrode potential (which may comprise the converted voltage) to its negative input. The subsequent filter may be realized by an exemplary 6-bit or n-bit digital integrator, whose output is then fed back to control the HV current sources.

Figure 3 shows an exemplary timing of the sampling phases for the control device of Fig. 2. The first switch 11 is set to "1", thus closed in the first half-cycle phase from t=0 to t=π to charge the capacitors C₁, C₂, while the second switch 12 is set to "0", thus open. In the second half-cycle phase from t= π to t=2π, the first switch 11 is open and the second switch 12 closed for a short time to reset the capacitive divider 10 into a known state. In order to maximize the accuracy of the ADC unit 5, the voltages are compared according to the shifted phase Φ_{0,d}, i.e. only when phase Φ_{0,d} is 1. The shifted phase Φ_{0,d} is delayed to compensate for a latency of, for instance, the first switch 11.

## Claims

1. Control device (1) for a reconfigurable current controlled/voltage controlled neural stimulation device (2) comprising
- an electrode voltage connector (3) configured to be connectable to the neural stimulation device (2) for obtaining an electrode voltage (V_{EL})of the neural stimulation device (2);
- a current control signal connector (4, 4') configured to be connectable to the neural stimulation device (2) for providing a current control signal (b[5:0], b[5], b[4:0]) to the neural stimulation device (2), where an electrode current of the neural stimulation device (2) is controlled by the current control signal (b[5:0], b[5], b[4:0]);
**characterized by**
- an analog-to-digital converter unit (5) configured to digitize an input voltage, which is the electrode voltage (V_{EL}) obtained via the electrode voltage connector (3) or a converted voltage corresponding to the electrode voltage (V_{EL}) obtained via the electrode voltage connector (3), relative to a preset reference voltage (V_{ref}), and to provide a digital output signal as a result of the digitization, which is a digitalization of the difference between the input voltage and the reference voltage;
- a digital control unit (6) configured to generate the current control signal (b[5:0], b[5], b[4:0]) in dependence upon the digital output signal provided by the ADC unit (5) and provide the current control signal (b[5:0], b[5], b[4:0]) to the current control signal connector (4, 4') so as to configure the current controlled neural stimulation device (2) into the voltage controlled neural stimulation device (2).

2. Control device (1) according to claim 1, **characterized in that** the preset reference voltage (V_{ref}) is a dynamically preset reference voltage.

3. Control device (1) according to any of the preceding claims, **characterized in that**
the preset reference voltage (V_{ref}) is selectable to be zero or another preset reference voltage by a user, and, in particular, the preset reference voltage (V_{ref}) is generated by the digital-to-analog unit (5).

4. Control device (1) according to any of the preceding claims, **characterized in that**
the analog-to-digital converter unit (5) comprises a 1-bit analog-to-digital converter (7) which is used in digitizing the input voltage relative to the reference voltage (V_{ref}).

5. Control device (1) according to any of the preceding claims, **characterized in that** the analog-to-digital converter unit (5) comprises a multi-bit analog-to-digital converter (7) which is used in digitizing the input voltage relative to the reference voltage (V_{ref}).

6. Control device (1) according to any of the preceding claims, **characterized by** a conversion unit (9) configured to convert the electrode voltage (V_{EL}) into the converted voltage as input voltage for the analog-to-digital converter unit (5), where the converted voltage is lower than the electrode voltage (V_{EL}).

7. Control device (1) according to claim 6, **characterized in that** the converted voltage is 5 Volt or lower, in particular 3 Volt or lower or 1,8 Volt or lower.

8. Control device (1) according to claim 6 or 7, **characterized in that** the conversion unit (9) comprises a capacitive divider (10), in particular a capacitive divider (10) with at least one variable capacity.

9. Control device (1) according to claim 8, **characterized by**
- the capacitive divider (10) being configured to periodically sample the electrode voltage (V_{EL}), and
- the analog-to-digital converter unit (5) being configured to periodically sample the input voltage with a phase (Φ_{0,d}) shifted, in particular delayed, with respect to the phase (Φ₀) of the sampling of the electrode voltage (V_{EL}).

10. Neural stimulation device (2) with a control device (1) according to any of the preceding claims.

11. Method for operating a control device (1) for a reconfigurable current controlled/voltage controlled neural stimulation device (2), comprising the method steps:
- obtaining an electrode voltage (V_{EL}) of the neural stimulation device (2);
- digitizing an input voltage, which is the obtained electrode voltage (V_{EL}) or a converted voltage corresponding to the obtained electrode voltage (V_{EL}) relative to a preset reference voltage (V_{ref});
- providing a digital output signal as a result of the digitization, which is a digitalization of the difference between the input voltage and the reference voltage;
- generating a current control signal (b[5:0], b[5], b[4:0]), which is to be used for controlling an electrode current of the neural stimulation device (2), in dependence upon the provided digital output signal; and
- providing the current control signal (b[5:0], b[5], b[4:0]) to the neural stimulation device (2) so as to configure the current controlled neural stimulation device into the voltage controlled neural stimulation device.

## Patentansprüche

1. Steuereinrichtung (1) für eine rekonfigurierbare stromgesteuerte/spannungsgesteuerte Neurostimulationseinrichtung (2), umfassend:
- einen Elektrodenspannung-Verbinder (3), der eingerichtet ist, mit der Neurostimulationseinrichtung (2) verbindbar zu sein, um eine Elektrodenspannung (V_{EL}) der Neurostimulationseinrichtung (2) zu erhalten;
- einen Stromsteuersignal-Verbinder (4, 4'), der eingerichtet ist, mit der Neurostimulationseinrichtung (2) verbindbar zu sein, um ein Stromsteuersignal (b[5:0], b[5], b[4:0]) für die Neurostimulationseinrichtung (2) bereitzustellen, wobei ein Elektrodenstrom der Neurostimulationseinrichtung (2) durch das Stromsteuersignal (b[5:0], b[5], b[4:0]) gesteuert ist;
**gekennzeichnet durch**
- eine Analog-Digital-Wandlereinheit (5), die eingerichtet ist, eine Eingangsspannung, die die über den Elektrodenspannung-Verbinder (3) erhaltene Elektrodenspannung (V_{EL}) ist oder eine umgewandelte Spannung, die der über den Elektrodenspannung-Verbinder (3) erhaltenen Elektrodenspannung (V_{EL}) entspricht, ist, relativ zu einer voreingestellten Referenzspannung (V_{ref}) zu digitalisieren, und ein digitales Ausgangssignal als ein Ergebnis der Digitalisierung, die eine Digitalisierung der Differenz zwischen der Eingangsspannung und der Referenzspannung ist, bereitzustellen;
- eine digitale Steuereinheit (6), die eingerichtet ist, das Stromsteuersignal (b[5:0], b[5], b[4:0]) in Abhängigkeit von dem digitalen Ausgangssignal, das von der AD-Wandlereinheit (5) bereitgestellt ist, zu erzeugen, und das Stromsteuersignal (b[5:0], b[5], b[4:0]) für den Stromsteuersignal-Verbinder (4, 4') bereitzustellen, um die stromgesteuerte Neurostimulationseinrichtung (2) in die spannungsgesteuerte Neurostimulationseinrichtung (2) zu konfigurieren.

2. Steuereinrichtung (1) nach Anspruch 1, **dadurch gekennzeichnet, dass**
die voreingestellte Referenzspannung (V_{ref}) eine dynamisch eingestellte Referenzspannung ist.

3. Steuereinrichtung (1) nach einem der vorangehenden Ansprüche,
**dadurch gekennzeichnet, dass**
die voreingestellte Referenzspannung (V_{ref}) durch einen Benutzer auswählbar ist, Null oder eine andere voreingestellte Referenzspannung zu sein, und insbesondere die voreingestellte Referenzspannung (V_{ref}) durch die Digital-Analog-Einheit (5) erzeugt ist.

4. Steuereinrichtung (1) nach einem der vorangehenden Ansprüche,
**dadurch gekennzeichnet, dass**
die Analog-Digital-Wandlereinheit (5) einen 1-Bit-Analog-Digital-Wandler (7) umfasst, der bei der Digitalisierung der Eingangsspannung relativ zur Referenzspannung (V_{ref}) genutzt wird.

5. Steuereinrichtung (1) nach einem der vorangehenden Ansprüche,
**dadurch gekennzeichnet, dass** die Analog-Digital-Wandlereinheit (5) einen Multibit-Analog-Digital-Wandler (7) umfasst, der bei der Digitalisierung der Eingangsspannung relativ zu der Referenzspannung (V_{ref}) genutzt wird.

6. Steuereinrichtung (1) nach einem der vorangehenden Ansprüche, **gekennzeichnet durch** eine Wandlereinheit (9), die eingerichtet ist, die Elektrodenspannung (V_{EL}) in die umgewandelte Spannung als Eingangsspannung für die Analog-Digital-Wandlereinheit (5) umzuwandeln, wobei die umgewandelte Spannung niedriger ist als die Elektrodenspannung (V_{EL}).

7. Steuereinrichtung (1) nach Anspruch 6, **dadurch gekennzeichnet, dass** die umgewandelte Spannung 5 Volt oder weniger beträgt, insbesondere 3 Volt oder weniger beträgt, oder 1,8 Volt oder weniger beträgt.

8. Steuereinrichtung (1) nach Anspruch 6 oder 7, **dadurch gekennzeichnet, dass** die Wandlereinheit (9) einen kapazitiven Teiler (10), insbesondere einen kapazitiven Teiler (10) mit zumindest einer variablen Kapazität, umfasst.

9. Steuereinrichtung (1) nach Anspruch 8, **gekennzeichnet durch**
- den kapazitiven Teiler (10), der eingerichtet ist, die Elektrodenspannung (V_{EL}) periodisch abzutasten, und
- wobei die Analog-Digital-Wandlereinheit (5) eingerichtet ist, die Eingangsspannung periodisch abzutasten mit einer Phase (Φ_{0,d}), die gegenüber der Phase (Φ₀) der Abtastung der Elektrodenspannung (V_{EL}) verschoben, insbesondere verzögert ist.

10. Neurostimulationseinrichtung (2) mit einer Steuereinrichtung (1) nach einem der vorangehenden Ansprüche.

11. Verfahren zum Betreiben einer Steuereinrichtung (1) für eine rekonfigurierbare stromgesteuerte/spannungsgesteuerte Neurostimulationseinrichtung (2), umfassend die Verfahrensschritte:
- Erhalten einer Elektrodenspannung (V_{EL}) der Neurostimulationseinrichtung (2);
- Digitalisieren einer Eingangsspannung, die die erhaltene Elektrodenspannung (V_{EL}) ist, oder einer umgewandelten Spannung entsprechend der erhaltenen Elektrodenspannung (V_{EL}) relativ zu einer voreingestellten Referenzspannung (V_{ref});
- Bereitstellen eines digitalen Ausgangssignals als ein Ergebnis der Digitalisierung, die eine Digitalisierung der Differenz zwischen der Eingangsspannung und der Referenzspannung ist;
- Erzeugen eines Stromsteuersignals (b[5:0], b[5], b[4:0]), das zum Steuern eines Elektrodenstroms der Neurostimulationseinrichtung (2) zu nutzen ist, in Abhängigkeit von dem bereitgestellten digitalen Ausgangssignal; und
- Bereitstellen des Stromsteuersignals (b[5:0], b[5], b[4:0]) für die Neurostimulationseinrichtung (2), um die stromgesteuerte Neurostimulationseinrichtung in die spannungsgesteuerte Neurosimulationseinrichtung zu konfigurieren.

## Revendications

1. Dispositif de commande (1) pour un dispositif de stimulation neurale reconfigurable commandé en tension/courant (2) comprenant
- un connecteur de tension d'électrode (3) configuré pour pouvoir être connecté au dispositif de stimulation neurale (2) afin d'obtenir une tension d'électrode (V_{EL}) du dispositif de stimulation neurale (2) ;
- un connecteur de signal de commande de courant (4, 4') configuré pour pouvoir être connecté au dispositif de stimulation neurale (2) afin d'envoyer un signal de commande de courant (b[5:0], b[5], b[4:0]) au dispositif de stimulation neurale (2), dans lequel un courant d'électrode du dispositif de stimulation neurale (2) est commandé par le signal de commande de courant (b[5:0], b[5], b[4:0]) ;
**caractérisé par**
- un convertisseur analogique-numérique (5) configuré pour numériser une tension d'entrée, qui est la tension d'électrode (V_{EL}) obtenue via le connecteur de tension d'électrode (3) ou une tension convertie correspondant à la tension d'électrode (V_{EL}) obtenue via le connecteur de tension d'électrode (3), par rapport à une tension de référence prédéfinie (V_{ref}), et pour envoyer un signal de sortie numérique en conséquence de la numérisation, qui est une numérisation de la différence entre la tension d'entrée et la tension de référence ;
- une unité de commande numérique (6) configurée pour générer le signal de commande de courant (b[5:0], b[5], b[4:0]) dépendant du signal de sortie numérique fourni par le convertisseur analogique-numérique (5) et envoyer le signal de commande de courant (b[5:0], b[5], b[4:0]) au connecteur de signal de commande de courant (4, 4') afin de configurer le dispositif de stimulation neurale commandé en courant (2) dans le dispositif de stimulation neural commandé en tension (2).

2. Dispositif de commande (1) selon la revendication 1, **caractérisé en ce que**
la tension de référence prédéfinie (V_{ref}) est une tension de référence prédéfinie de manière dynamique.

3. Dispositif de commande (1) selon l'une quelconque des revendications précédentes, **caractérisé en ce que**
la tension de référence prédéfinie (V_{ref}) peut être sélectionnée sur zéro ou sur une autre tension de référence prédéfinie par un utilisateur, et, notamment, la tension de référence prédéfinie (V_{ref}) est générée par le convertisseur analogique-numérique (5).

4. Dispositif de commande (1) selon l'une quelconque des revendications précédentes, **caractérisé en ce que**
le convertisseur analogique-numérique (5) comprend un convertisseur analogique-numérique 1 bit (7) qui sert à numériser la tension d'entrée par rapport à la tension de référence (V_{ref}).

5. Dispositif de commande (1) selon l'une quelconque des revendications précédentes, **caractérisé en ce que** le convertisseur analogique-numérique (5) comprend un convertisseur analogique-numérique multi-bit (7) qui sert à numériser la tension d'entrée par rapport à la tension de référence (V_{ref}).

6. Dispositif de commande (1) selon l'une quelconque des revendications précédentes, **caractérisé par** un convertisseur (9) configuré pour convertir la tension d'électrode (V_{EL}) vers la tension convertie comme tension d'entrée pour le convertisseur analogique-numérique (5), où la tension convertie est inférieure à la tension d'électrode (V_{EL}).

7. Dispositif de commande (1) selon la revendication 6, **caractérisé en ce que** la tension convertie est de 5 volts ou inférieure, notamment de 3 volts ou inférieure ou de 1,8 volts ou inférieure.

8. Dispositif de commande (1) selon les revendications 6 ou 7, **caractérisé en ce que** le convertisseur (9) comprend un diviseur capacitif (10), notamment un diviseur capacitif (10) avec au moins une capacité variable.

9. Dispositif de commande (1) selon la revendication 8, **caractérisé par**
- le diviseur capacitif (10) étant configuré pour échantillonner périodiquement la tension d'électrode (V_{EL}), et
- le convertisseur analogique-numérique (5) étant configuré pour échantillonner périodiquement la tension d'entrée avec un changement de phase (Φ_{0,d}), notamment un retard, par rapport à la phase (Φ₀) de l'échantillonnage de la tension d'électrode (V_{EL}).

10. Dispositif de stimulation neurale (2) avec un dispositif de commande (1) selon l'une quelconque des revendications précédentes.

11. Méthode d'utilisation d'un dispositif de commande (1) pour un dispositif de stimulation neurale reconfigurable commandé en tension/courant (2), comprenant les étapes suivantes :
- obtention d'une tension d'électrode (V_{EL}) du dispositif de stimulation neurale (2) ;
- numérisation d'une tension d'entrée, qui est la tension d'électrode obtenue (V_{EL}) ou une tension convertie correspondant à la tension d'électrode obtenue (V_{EL}) par rapport à une tension de référence prédéfinie (V_{ref}) ;
- envoi d'un signal de sortie numérique en conséquence de la numérisation, qui est une numérisation de la différence entre la tension d'entrée et la tension de référence ;
- génération d'un signal de commande de courant (b[5:0], b[5], b[4:0]), qui doit être utilisé pour commander un courant d'électrode du dispositif de stimulation neurale (2), dépendant du signal de sortie numérique fourni ; et
- envoi du signal de commande de courant (b[5:0], b[5], b[4:0]) vers le dispositif de stimulation neurale (2) afin de configurer le dispositif de stimulation neurale commandé en courant dans le dispositif de stimulation neurale commandé en tension.
